# FASCICULE DE BREVET EUROPEEN

(11) **EP 2 105 119 B1**
(45) Date de publication et mention de la délivrance du brevet: **15.06.2016**
(21) Numéro de dépôt: 09290209.7
(22) Date de dépôt: 20.03.2009
(51) Int. Cl.: A61K 8/02, A61K 8/26, A61K 8/97, A61Q 11/00

(54) **Dentifrice en poudre fluide et procédé de réalisation correspondant**
Zahnpasta aus Flüssigpulver und entsprechendes Herstellungsverfahren
Liquid powder toothpaste and corresponding production method

(30) Priorité: 25.03.2008 FR 0801595
(43) Date de publication de la demande: 30.09.2009
(73) Titulaire: Esprit d'Ethique, 44700 Orvault (FR)
(72) Inventeur: Binachon, Christophe, 44700 Orvault (FR)
(74) Mandataire: Lavialle, Bruno François Stéphane

(56) Documents cités:
- DE-A1- 19 961 936
- GB-A- 485 188
- GB-A- 544 405
- US-A1- 2003 068 358
- US-B1- 6 379 654
- US-B1- 6 503 541
- US-B1- 6 645 472
- ANONYME: "Papaya" INTERNET, [Online] 18 décembre 2007 (2007-12-18), XP002514872 Extrait de l'Internet: URL:http://web.archive.org/web/20071218104 358/http://www.buy-vitamins-minerals.com/p apaya.htm> [extrait le 2009-02-10]
- DAWSON E: "The medicinal properties of the papaya, Carica papaya L" INTERNET CITATION, [Online] XP002271935 Extrait de l'Internet: URL:www.siu.edu/bl/leaflets/papaya.htm> [extrait le 2004-02-18]

## Description

La présente invention concerne un dentifrice en poudre fluide pour le traitement des gencives.

### ARRIERE PLAN DE L'INVENTION

On sait que les extraits de feuille de différentes plantes ont des vertus anti-inflammatoires sur les gencives.

On sait également que les extraits de feuilles sont plus actifs lorsqu'ils sont appliqués sous forme de poudre. Toutefois, les extraits de feuilles en poudre sont très hydrophiles de sorte que lorsqu'un extrait de feuilles est conservé sous forme d'une poudre, les grains s'agglomèrent très rapidement pour former des blocs qui sont inutilisables avec une brosse à dents.

On connait par ailleurs du document FR 2 785 534 un procédé de réalisation d'une composition abrasive pour dentifrice comprenant les étapes de réaliser une solution aqueuse de grains de matériau calcique et d'un sel d'acide carboxylique, et de sécher la solution, notamment par atomisation. Le produit obtenu n'a aucune vertu de traitement des gencives.

### OBJET DE L'INVENTION

Un but de l'invention est de proposer un dentifrice en poudre fluide pour le traitement des gencives conservant une bonne fluidité même après un stockage prolongé, ainsi que le procédé de réalisation correspondant.

### RESUME DE L'INVENTION

En vue de la réalisation de ce but, on propose selon l'invention un dentifrice en poudre comportant des grains de pierre ponce broyée enrobés de particules d'un extrait sec de feuille d'arbre fruitier en particulier du papayer.

Il a en effet été constaté que la fixation des particules d'extrait sec de feuille de papayer sur des grains de pierre ponce broyée assurait un maintien du dentifrice en poudre dans un état fluide, même après un temps de conservation important. En outre, il a été constaté que l'action mécanique douce des grains de pierre ponce a un effet de synergie avec des propriétés de l'extrait sec de feuille de papayer de sorte que le dentifrice ainsi réalisé a des effets supérieurs à ceux obtenus avec un extrait actif pur ou même un mélange d'un extrait actif en poudre et d'un produit minéral ou organique en grains.

De préférence, les grains de pierre ponce broyée ont une dimension maximale de 100 µm et ont en majorité une dimension inférieure ou égale à 50 µm.

Pour la réalisation de ce dentifrice, on propose selon l'invention un procédé comportant les étapes :
- de réaliser une décoction de feuilles de papayer dans de l'eau et d'extraire le liquide obtenu,
- d'ajouter des grains de pierre ponce broyée au liquide obtenu,
- d'atomiser le mélange dans un courant d'air chaud.

Il a été constaté que l'utilisation de la pierre ponce permet d'obtenir un mélange exceptionnellement homogène avant l'atomisation, ce qui favorise une bonne exécution de cette étape.

### DESCRIPTION DETAILLEE DE L'INVENTION

Selon un mode de mise en oeuvre préféré de l'invention, des feuilles sèches de papayer sont mises à tremper dans un poids d'eau égal à environ trente fois le poids de feuilles sèches. L'ensemble est maintenu à 90°C pendant une heure avec agitation. Une extraction de liquide est effectuée en passant tout d'abord la décoction sur une grille à mailles de 8 mm puis dans un filtre à mailles de 100 µm.

Un échantillon de décoction est soumis à un évaporateur à air chaud dont la température d'entrée est voisine de 300°C et dont la température de sortie est voisine de 100°C afin de déterminer la quantité de matière sèche contenue dans la décoction finale.

De la pierre ponce finement broyée dont les grains ont une dimension maximale de 100 µm et majoritairement une dimension inférieure ou égale à 50 µm, est ajoutée dans la décoction à raison de une fois et demi en poids de la matière sèche contenue dans la décoction.

Le mélange est soumis à un évaporateur à air chaud dont la température d'entrée est voisine de 300°C et la température de sortie est voisine de 100°C. La turbine de dispersion est entraînée à une vitesse de rotation élevée afin d'obtenir une dispersion fine du produit dans l'enceinte de séchage.

La poudre sèche obtenue a été analysée au microscope électronique, ce qui a révélé que les grains de feuille de papayer enrobent les grains de pierre ponce auxquels ils sont attachés. La poudre obtenue est fluide et peut aisément être utilisée en humidifiant une brosse à dents et en procédant à un nettoyage des dents et des gencives selon un usage habituel d'une brosse à dent. Une expérimentation a révélé des propriétés anti-inflammatoires du dentifrice en poudre ainsi utilisé.

Bien entendu, l'invention n'est pas limitée au mode de mise en oeuvre particulier décrit et on peut y apporter des variantes de réalisation sans sortir du cadre de l'invention tel que défini par les revendications.

En particulier, l'extraction de la matière active en une seule étape en mettant les feuilles de papayer sèches à tremper dans environ trente fois le poids de feuilles sèches peut être remplacée par une extraction en plusieurs étapes permettant une extraction plus complète des principes actifs contenus dans les feuilles de papayer. A titre d'exemple, les feuilles de papayer sèches sont mise à tremper une première fois dans un poids d'eau égal à environ vingt fois le poids de feuilles sèches et une première extraction de liquide est effectuée dans les conditions décrites ci-dessus; les feuilles de papayer sont à nouveau mises à tremper cette fois dans un poids d'eau égal à environ 2,5 fois le poids de la matière sèche de départ. L'ensemble est maintenu à 90°C pendant dix minutes sans agitation puis est filtré comme précédemment tout d'abord avec un filtre à mailles de 8 mm puis avec un filtre à mailles de 100 µm. Le liquide obtenu est mélangé avec le liquide obtenu lors de la première extraction.

Préalablement à l'évaporation on peut en outre prévoir une concentration du liquide extrait dans un concentrateur sous vide à 110°C jusqu'à obtenir 1/10ème de la décoction de départ.

La décoction peut également être réalisée à partir de feuilles fraîches, la quantité d'eau étant diminuée dans des proportions correspondantes, c'est à dire que les feuilles fraîches sont mises à tremper dans un poids d'eau égal à environ six fois le poids des feuilles frâiches.

Bien que dans le procédé de mise en oeuvre préféré décrit il soit prévu de mettre une quantité de poudre de pierre ponce égale à une fois et demi en poids la quantité d'extrait sec de la décoction, ce qui correspond à un enrobage complet des grains de pierre ponce par l'extrait de feuille de papayer, on peut également prévoir de réaliser un dentifrice moins actif en introduisant par exemple de la pierre ponce à raison de deux fois le poids estimé d'extrait sec dans la décoction initiale.

Il est également possible de réaliser un dentifrice en poudre plus actif en introduisant la matière support à raison de une fois seulement le poids d'extrait sec dans la décoction initiale. Dans ce cas, l'analyse au microscope électronique révèle des grains d'extrait sec de feuilles de papayer non supportés par des grains de pierre ponce de sorte que le dentifrice obtenu doit être conservé dans un endroit sec afin d'éviter une agglomération des grains sous forme de blocs qui seraient inutilisables.

Bien que l'invention ait été décrite en relation avec un extrait actif obtenu par une décoction de feuilles de papayer dans de l'eau, on peut mettre en oeuvre le procédé de l'invention en relation avec d'autres extraits actifs naturels, par exemple une décoction dans de l'eau de feuilles de framboisier ou de théier.

D'autres composants, par exemple des agents de blanchiment tels que décrits dans le document mentionné en préambule peuvent également être ajoutés dans le dentifrice de l'invention.

## Revendications

1. Dentifrice en poudre, **caractérisé en ce qu'**il comporte des grains de pierre ponce broyée enrobés de particules d'extrait sec de feuilles d'arbre fruitier.

2. Dentifrice en poudre selon la revendication 1, **caractérisé en ce que** les grains du support ont une dimension maximale de 100 µm et ont en majorité une dimension inférieure ou égale à 50 µm.

3. Procédé de réalisation d'un dentifrice en poudre, **caractérisé en ce qu'**il comporte les étapes:
- de réaliser une décoction de feuilles d'arbre fruitier dans de l'eau et d'extraire le liquide obtenu,
- d'ajouter des grains de pierre ponce broyée au liquide obtenu,
- d'atomiser le mélange dans un courant d'air chaud.

4. Procédé selon la revendication 3, **caractérisé en ce que** le support en grains est ajouté selon une quantité en poids comprise entre une à deux fois, et de préférence une fois et demi, une quantité en poids d'extrait sec dans la décoction filtrée.

5. Procédé selon la revendication 3, **caractérisé en ce que** des feuilles sèches d'arbre fruitier sont mises à tremper dans un poids d'eau égal à trente fois le poids de feuilles sèches.

6. Procédé selon la revendication 3, **caractérisé en ce que** la décoction est filtrée avec un tamis ayant une maille de 100 µm.

7. Procédé selon la revendication 3, **caractérisé en ce que** les grains de pierre ponce ont une dimension maximale égale à 100 µm et ont en majorité une dimension inférieure ou égale à 50 µm.

8. Procédé selon la revendication 3, **caractérisé en ce que** le mélange est atomisé dans un courant d'air ayant une température d'entrée voisine de 300°C et une température de sortie voisine de 100°C.

## Patentansprüche

1. Zahnpasta in Pulverform, **dadurch gekennzeichnet, dass** sie Körner aus zerkleinertem Bimsstein umfasst, die von Trockenextraktpartikeln aus Obstbaumblättern umhüllt sind.

2. Zahnpasta in Pulverform nach Anspruch 1, **dadurch gekennzeichnet, dass** die Körner des Trägers eine maximale Abmessung von 100 µm und mehrheitlich eine Abmessung haben, die kleiner oder gleich 50 µm ist.

3. Verfahren zur Herstellung einer Zahnpasta in Pulverform, **dadurch gekennzeichnet, dass** es die Schritte umfasst:
- Herstellen eines Dekokts aus Obstbaumblättern in Wasser und Extrahieren der erhaltenen Flüssigkeit,
- Hinzufügen von Körnern aus zerkleinertem Bimsstein zur erhaltenen Flüssigkeit,
- Zerstäuben der Mischung in einem Warmluftstrom.

4. Verfahren nach Anspruch 3, **dadurch gekennzeichnet, dass** der Träger aus Körnern in einer Gewichtsmenge hinzugefügt wird, die zwischen dem Ein- bis Zweifachen und vorzugsweise bei dem Eineinhalbfachen einer Gewichtsmenge an Trockenextrakt in dem gefilterten Dekokt liegt.

5. Verfahren nach Anspruch 3, **dadurch gekennzeichnet, dass** die trockenen Blätter des Obstbaums in einem Gewicht an Wasser gleich dem Dreißigfachen des Gewichts an trockenen Blättern eingeweicht werden.

6. Verfahren nach Anspruch 3, **dadurch gekennzeichnet, dass** das Dekokt mit einem Sieb gefiltert wird, das eine Lochweite von 100 µm hat.

7. Verfahren nach Anspruch 3, **dadurch gekennzeichnet, dass** die Bimssteinkörner eine maximale Abmessung haben, die gleich 100 µm ist, und mehrheitlich eine Abmessung haben, die kleiner oder gleich 50 µm ist.

8. Verfahren nach Anspruch 3, **dadurch gekennzeichnet, dass** die Mischung in einem Luftstrom zerstäubt wird, der eine Eintrittstemperatur nahe 300°C und eine Austrittstemperatur nahe 100°C hat.

## Claims

1. A dentifrice powder, comprising grains of ground pumice stone coated with particles of dry extract of fruit tree leaves.

2. A dentifrice powder according to claim 1, wherein the maximum dimension of the support grains is 100 µm and the majority of them have a dimension of 50 µm or less.

3. A method of producing a dentifrice powder, comprising the following steps:
• producing a decoction of fruit tree leaves in water and extracting the liquid obtained;
• adding grains of ground pumice stone to the liquid obtained;
• atomizing the mixture in a stream of hot air.

4. A method according to claim 3, wherein the support in the form of grains is added in a quantity by weight in the range from one to two times, preferably one and one-half times the quantity by weight of the dry extract in the filtered decoction.

5. A method according to claim 3, wherein the dry fruit tree leaves are soaked in a weight of water equal to thirty times the weight of the dry leaves.

6. A method according to claim 3, wherein the decoction is filtered with a sieve with a mesh of 100 µm.

7. A method according to claim 3, wherein the grains of pumice stone have a maximum dimension of 100 µm and the majority have a dimension of 50 µm or less.

8. A method according to claim 3, wherein the mixture is atomized in a stream of air having an inlet temperature of close to 300°C and an outlet temperature of close to 100°C.
